Europäisches Patentamt
European Patent Office
Office européen des brevets

(19) (11) Publication number: 0 001 041
A2

(12) **EUROPEAN PATENT APPLICATION**

(21) Application number: 78100408.0

(22) Date of filing: 17.07.78

(51) Int. Cl.[2]: C 07 D 499/64
C 07 D 501/32, C 07 D 501/22
C 07 D 213/80, C 07 D 213/82
A 61 K 31/43, A 61 K 31/545

(30) Priority: 27.07.77 CH 9280/77

(43) Date of publication of application: 21.03.79 Bulletin 79/6

(84) Designated contracting states: BE CH DE FR GB LU NL SE

(71) Applicant: SANDOZ LTD.
Lichtstrasse 35
CH-4002 Basel(CH)

(72) Inventor: Ascher, Gerd, Dr.
Karl Schwedgasse 113
A-1138 Vienna(AT)

(72) Inventor: Hamberger, Helmut, Dr.
Josef-Kyrlegasse 4-6
A-1130 Vienna(AT)

(72) Inventor: Stütz, Peter, Dr.
Nästlbergergasse 15
A-1130 Vienna(AT)

(54) **Alpha(pyridonecarboxylamino)-alpha substituted acetamido penicillins and -cephalosporins, process for their preparation, their use in pharmaceuticals; pyridones used in the process.**

(57) α(Pyridonecarbonylamino)-α- substituted acetamido penicillins and –cephalosporins, process for their preparation, their use in pharmaceuticals and pyridones used in the process.
The compounds are of formula I:

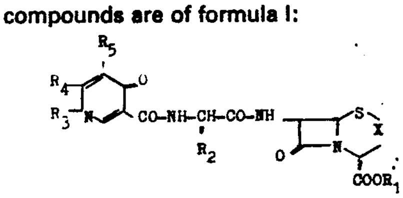

(I)

in which X is 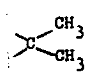 or 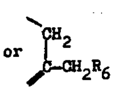

The pyridines of formulas IV and VI are also described:

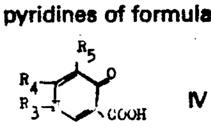

IV

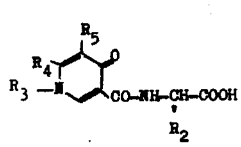

VI

EP 0 001 041 A2

Case 900-9194

0001041

TITLE MODIFIED
see front page

# IMPROVEMENTS IN OR RELATING TO ORGANIC COMPOUNDS

This invention relates to novel penicillins and cephalosporins.

More particularly, this invention provides compounds of formula I,

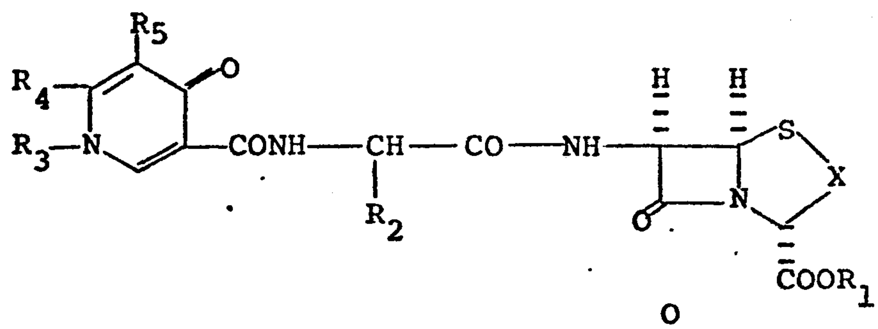

in which $R_1$ is hydrogen or $-CH_2O\overset{O}{\overset{\|}{C}}C(CH_3)_3$,

$R_2$ is phenyl, 4-hydroxyphenyl or 1,4-cyclohexadien-1-yl,

$R_3$ is lower alkyl, unsubstituted or mono- or poly-substituted by alkoxy; allyl; cyclohexyl; cyclopropylmethyl; or benzyl or phenyl, such benzyl or phenyl being unsubstituted or mono- or poly-substituted in the phenyl ring by lower alkyl, lower alkoxy, lower alkoxycarbonyl, cyano, trifluoromethyl, chlorine or bromine,

$R_4$ is hydrogen, cyano, lower alkyl or hydroxy,

$R_5$ is hydrogen, chlorine or bromine,

and X is a group of formula IIa or IIb,

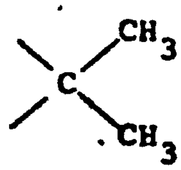

IIa

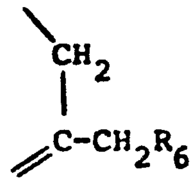

IIb

in which $R_6$ is hydrogen or acetoxy,

and salts of compounds of formula I, in which $R_1$ is hydrogen.

The invention also provides processes for the production of compounds of formula I, comprising

a) reacting a compound of formula III,

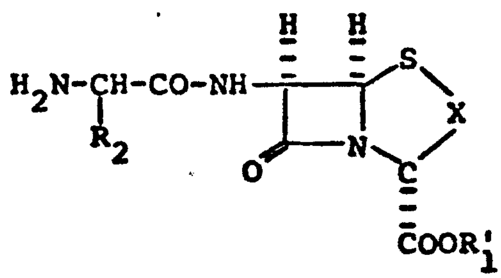

III

in which $R_2$ and X are as defined above, and

$R_1'$ is hydrogen, $-CH_2O\overset{O}{\overset{\|}{C}}C(CH_3)_3$, or a carboxylic acid protecting group removable under mild conditions,

with a carboxylic acid of formula IV,

BAD ORIGINAL

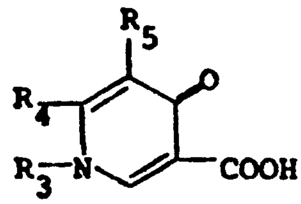

IV

in which $R_3$, $R_4$ and $R_5$ are as defined above, or a reactive derivative thereof, and, where $R_1'$ signifies a carboxylic acid protecting group, removing this under mild conditions,

or b) reacting a compound of formula V,

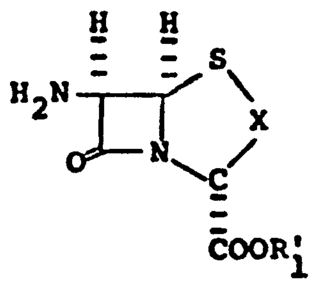

V

in which $R_1'$ and X are as defined above, with a carboxylic acid of formula VI,

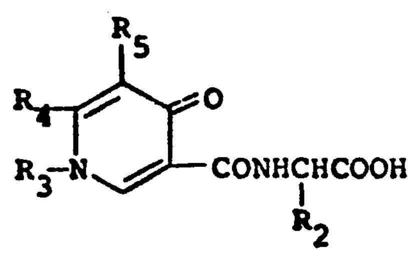

VI

in which $R_2$, $R_3$, $R_4$ and $R_5$ are as defined above, or a reactive derivative thereof, and, where $R_1'$ signifies a carboxylic acid protecting group, removing this under mild conditions.

Processes a) and b) may be carried out in similar and conventional manner. For example, the compound of formula IV or VI, or a reactive derivative thereof, e.g. an acid halide, acid anhydride or ester thereof, may be dissolved or suspended in an inert solvent, for example a cyclic ether, such as tetrahydrofuran, or a chlorinated hydrocarbon, such as chloroform or methylene chloride, and mixed with a solution of a compound of formula III or V in the same solvent. Where the compound of formula IV or VI is employed in free acid form, a condensation agent, such as dicyclohexylcarbodiimide or carbonyldiimidazole, is suitably added to the reaction mixture. The reaction mixture is then suitably stirred at a temperature of, for example, -30°C to +30°C. Suitable carboxylic acid protecting groups $R_1'$ are well known in the penicillin/cephalosporin field and include benzhydryl and trialkylsilyl groups. Such protecting groups may be removed in conventional manner.

The resulting compounds of formula I may be isolated and purified using conventional techniques. Salt forms of the compounds of formula I, in which $R_1$ is hydrogen, may be produced either by employing the starting material of formula IV or VI on the corresponding salt form or by converting a resulting compound of formula I, in which $R_1$ is hydrogen, in conventional manner to the salt forms. Suitable salt forms include alkali metal salts, in particular potassium salts.

The compounds of formula IV are also new and may, for example, be produced by

c) obtaining a compound of formula IVa,

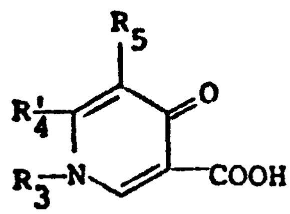

IVa

in which $R_3$ and $R_5$ are as defined above, and

$R_4'$ is hydrogen or lower alkyl,

by reacting a compound of formula VII,

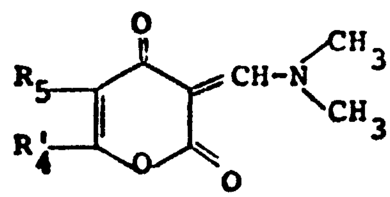

VII

in which $R_4'$ and $R_5$ are as defined above,

with a compound of formula VIII,

$$R_3\text{-}NH_2 \qquad \text{VIII}$$

in which $R_3$ is as defined above,

or d) obtaining a compound of formula IVb,

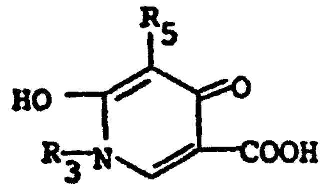

IVb

in which $R_3$ and $R_5$ are as defined above,

desulphurating a compound of formula IX,

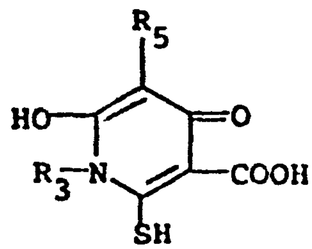

IX

in which $R_3$ and $R_5$ are as defined above.

These processes may be carried out in conventional manner, the desulphuration in process d) for example being effected with e.g. Raney Nickel. Other methods of producing compounds of formula IV are also illustrated in the examples hereinafter, in particular various interconversion methods.

The compounds of formula VI are also new and may for example be produced by reacting a compound of formula IV or a reactive derivative thereof, with a compound of formula X,

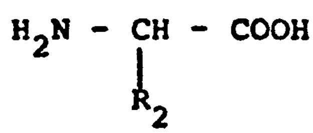

X

in which $R_2$ is as defined above,

in the form, for example, of its triethylammonium salt.

This process is suitably effected in an inert solvent, such as chlorinated hydrocarbon, e.g. methylene chloride or chloroform, or dimethylformamide. When the compound of formula IV is employed in free acid form, a

condensation agent, e.g. dicyclohexylcarbodiimide, is suitably employed.

The resulting compounds of formula IV and VI may be isolated and purified in conventional manner. Where required, reactive derivatives thereof may be produced in conventional manner from the free acids.

The compounds of formula XI, employed as starting materials in process d) may be produced by reacting, in conventional manner, a compound of formula XI,

$$R_6\text{-OOC-CH}(R_5)\text{-}\overset{O}{\overset{\|}{C}}\text{-CH}_2\text{-COO-}R_7 \qquad XI$$

in which $R_5$ is as defined above, and

$R_6$ and $R_7$ are each lower alkyl,

with an isothiocyanate of formula XII,

$$R_3 - N = C = S \qquad XII$$

in which $R_3$ is as defined above.

The compounds of formula I are useful as pharmaceuticals. In particular, they are useful as antimicrobial agents, more particularly as anti-bacterial antibiotics, as indicated <u>in vitro</u> in the series dilution test at concentrations of, for example, 0.1 to 50 µg/ml, and <u>in vivo</u> in standard tests in mice at a dosage of, for example 30 to 150 mg/kg of animal body weight, against a variety of

bacterial strains, for example Strept. pyogenes, E. coli, Proteus vulgaris, mirabilis and morganii, Shigella flexneri, Serratia marcescens, Neisseria subflava, Salmonella, Pasteurella multocida, Haemophilus influenzae, Haemophilus sp., Clostridium perfringens, Bacteroides fragilis and melaninogenicus, Fusobacterium fusiforme and Sphaerophorus necrophorus.

An indicated suitable total daily dosage is from 1 to 10 g, suitably administered in divided doses of from 0.25 to 5 g, two to four times daily, or in retard form.

The compounds may be admixed with conventional pharmaceutically acceptable diluents or carriers and, optionally, other excipients, and administered in such forms as capsules, or injectable or drop preparations.

The compounds of formula I, in which $R_1$ is hydrogen may be employed in free acid form or in the form of

pharmaceutically acceptable salts, which salt forms have the same order of activity as the free acid forms. Suitable salts include alkali metal, e.g. potassium, salts.

The compounds of formula I fall into two broad classes, namely penicillins (X is IIa) and cephalosporins (X is IIb) of which the penicillins are preferred. In the cephalosporins $R_6$ may be hydrogen. Alternatively, it may be acetoxy.

$R_1$ may be hydrogen; alternatively it may be pivaloyloxymethyl. $R_2$ may be phenyl. Alternatively, it may be 1,4-cyclohexadien-1-yl. Preferably, it is p-hydroxyphenyl. $R_4$ may be hydrogen. Alternatively it may be lower alkyl. Finally, it may be hydroxy. $R_5$ is suitably hydrogen. Alternatively, it may be chlorine or, preferably, bromine.

$R_3$ can be lower alkyl. This can be unsubstituted. Alternatively, it can be mono- or poly- (e.g. di-) substituted by alkoxy, in particular lower alkoxy, e.g. 2,2-diethoxyethyl. $R_3$ can also be allyl; cyclohexyl; or cyclopropylmethyl. It may also be phenyl or benzyl. These may be unsubstituted; or they may be mono- or poly- (e.g. di- or tri-) substituted by lower alkyl, lower alkoxycarbonyl, cyano, trifluoromethyl, chlorine or bromine, in particular by lower alkyl, lower alkoxy or halogen, e.g. chlorine, more particularly mono-substituted by chlorine or methyl. $R_3$ is preferably ethyl, benzyl or allyl

The term "lower" used herein in connection with alkyl and alkoxy groups means containing 1 to 4, preferably 1 to 2 carbon atoms.

The following Examples illustrate the invention, all temperatures being in degrees Centigrade.

**EXAMPLE 1:** Pivaloyloxymethyl 6-{α-[1-ethyl-6-methyl-(4)-pyridone-3-carbonylamino]phenylacetamido}-penicillanate

A suspension of 1.8 g of 1-ethyl-6-methyl-(4)-pyridone-3-carboxylic acid in 50 ml of tetrahydrofuran is mixed, under argon, at 0° with 1.4 ml of triethylamine. 1.08 g of ethyl chloroformate in 10 ml of tetrahydrofuran are added, dropwise, the reaction mixture is stirred for 1 hour at 0° and the resulting precipitate is filtered off. 5 g of pivampicillin hydrochloride are dissolved in 100 ml of water, 0.84 g of sodium bicarbonate are added and the mixture is extracted with ethyl acetate. The organic phase is dried over sodium chloride, evaporated *in vacuo*, taken up in 25 ml of tetrahydrofuran and slowly added, dropwise, to the filtrate obtained above. The reaction mixture is stirred for 1 hour at room temperature, evaporated *in vacuo*, and the residue taken up in chloroform. The mixture is washed with water, then with 0.42 g of sodium bicarbonate in 70 ml of water, again with water and then with 5 ml of 1N hydrogen chloride in 100 ml of water and finally again with water. The chloroform phase is dried over sodium sulphate and evaporated *in vacuo*. The residue is taken up in 10 ml of ethyl acetate and the product is crystallised by seeding, filtered and dried *in vacuo*.
M.P. 203° (decomp.).

EXAMPLE 2: Potassium 6-{α-[1-ethyl-6-methyl-(4)-pyridone-3-carbonylamino]phenylacetamido}penicillanate

A suspension of 0.36 g of 1-ethyl-6-methyl-(4)-pyridone-3-carboxylic acid in 10 ml of chloroform is mixed, under argon, at 0° with 0.28 ml of triethylamine. 0.2 ml of ethyl chloroformate in 2 ml of chloroform are slowly added, dropwise, and the reaction mixture is stirred at 0° for 1 hour. 0.81 g of ampicillin trihydrate are stirred in 15 ml of chloroform at room temperature with 0.6 g of magnesium sulphate and 0.42 ml of triethylamine for 1 hour. After filtration of the magnesium sulphate, the filtrate is slowly added, dropwise, at 20° to the above solution and, after addition, the mixture is stirred at room temperature for 3 hours. The reaction mixture is evaporated *in vacuo*, and the residue is taken up in 15 ml of water and 0.16 g of sodium bicarbonate and then shaken with chloroform. The aqueous phase is saturated with sodium chloride, acidified with 1N hydrochloric acid to pH 1.5, and extracted with chloroform. The chloroform phase is dried and evaporated *in vacuo*, triturated with ether, filtered and dried. The resulting precipitate is suspended in 20 ml of water, dissolved by addition of 0.19 g of potassium bicarbonate and freeze dried. M.p. 207-209° (decomp.).

EXAMPLE 3: Potassium 6-{α-[1-ethyl-6-methyl-(4)-pyridone-3-carbonylamino]phenylacetamido}penicillanate

A suspension of 0.36 g of 1-ethyl-6-methyl-(4)-pyridone-3-carboxylic acid in 10 ml of ethanol-3-chloroform is mixed, under argon, at 0° with 0.28 ml of triethylamine. 0.2 ml of ethyl chloroformate in 2 ml of chloroform are added, dropwise, slowly and the reaction mixture is stirred for 1 hour at 0°. 0.81 g of ampicillin trihydrate are suspended in 3 ml of water and 2 ml of 1N sodium hydroxide, 0.17 g of sodium bicarbonate, and 20 mg of benzyltriethylammonium chloride are added successively. The 2 solutions obtained above are mixed and stirred at room temperature overnight. The aqueous phase is acidified to pH 2 with 1N hydrochloric acid, saturated with sodium chloride and extracted with chloroform. After drying and evaporation *in vacuo*, the residue is rubbed with ether, filtered and dried. The precipitate is suspended in 10 ml of water, dissolved by addition of 0.13 g of potassium bicarbonate and freeze dried. M.p. 207-209° (decomp.).

EXAMPLE 4: Pivaloyloxymethyl 6-{α-[1-allyl-6-methyl-(4)-pyridone-3-carbonylamino]phenylacetamido}-penicillanate

In manner analogous to Example 1 and employing appropriate starting materials in approximately equivalent

amounts, the heading compound, m.p. 99-101°, may be obtained.

EXAMPLE 5: 6-{α-[1-allyl-6-methyl-(4)-pyridone-3-carbonylamino]phenylacetamido}penicillanic acid

0.39 g of 1-allyl-6-methyl-(4)-pyridone-3-carboxylic acid in 10 ml of ethanol-free-dichloromethane are converted in manner analogous to that in Example 3, at 0° to the mixed anhydride. 0.81 g of ampicillin trihydrate in converted, in manner analogous to that in Example 2, into water-free form and added slowly, dropwise, at 20° to the above solution. After a reaction time of 5 hours at room temperature and working up in accordance to the procedure described in Example 2, the heading compound, m.p. 236° (decomp.) is obtained in the form of its potassium salt.

EXAMPLE 6: Pivaloyloxymethyl 6-{α-[1-ethyl-6-hydroxy-(4)-pyridone-3-carbonylamino]phenylacetamido}-penicillanate

1 g of pivampicillin hydrochloride in 15 ml of dichloromethane is converted in ethyl acetate into the base form as described in Example 1. A suspension of 0.37 g of 1-ethyl-6-hydroxy-(4)-pyridone-3-carboxylic acid in 7 ml of dichloromethane is mixed, under argon, at 0° with 0.28 ml of triethylamine. The mixture is then mixed, dropwise, with 0.147 ml of thionyl chloride in 2 ml of dichloromethane. The reaction mixture is stirred at room temperature for 30 minutes. This suspension and a solution of 0.42 ml of triethylamine in 5 ml of dichloromethane are simultaneously added at -20° to the above-obtained pivampicillin solution, dropwise. The reaction mixture is stirred overnight at -20°, taken up in chloroform after careful evaporation and washed successively with water, 0.084 g of sodium bicarbonate in 20 ml of water, water, 1 ml of 1N hydrochloric acid in 20 ml of water and again with water. The chloroform phase is dried over sodium sulphate and evaporated in vacuo. The residue is rubbed with ether, filtered and dried in vacuo. M.p. 126-128°.

EXAMPLE 7: Potassium 6-{α-[1-ethyl-6-hydroxy-(4)-pyridone-3-carbonylamino]phenylacetamido}penicillanate

0.81 mg of ampicillin trihydrate in dichloromethane are dehydrated in analogous manner to Example 2 and mixed simultaneously with 0.42 ml of triethylamine and a solution of the acid chloride of 0.37 g of 1-ethyl-6-hydroxy-(4)-pyridone-3-carboxylic acid (obtained in accordance with Example 6), dropwise, at -30°. The reaction mixture is stirred overnight at -20° and stirred for a further 30 minutes at room temperature. After evaporation of the

organic phase. The residue is carefully mixed with 10 ml of 0.5 N hydrochloric acid (pH = 1.5) and the resulting precipitate is filtered, washed with water and dried. 0.66 g of the precipitate are suspended in 20 ml of water, dissolved by addition of 130 mg of potassium bicarbonate and freeze dried. M.p. 275° (decomp.).

<u>EXAMPLE 8</u>: <u>7-{α-[1-ethyl-6-methyl-(4)-pyridone-3-carbonylamino]phenylacetamido}cephalosporanic acid</u>

A suspension of 1.8 g of 1-ethyl-6-methyl-(4)-pyridone-3-carboxylic acid in 50 ml of tetrahydrofuran is mixed, under argon, at 0° with 1.4 ml of triethylamine. 0.95 ml of ethyl chloroformate in 10 ml of tetrahydrofuran are slowly added, dropwise, and the reaction mixture is stirred for 1 hour at 0°. After filtration of the precipitate, 4.39 g of 7-aminocephalosporanic acid benzhydryl ester in 20 ml of tetrahydrofuran are added, at 0°, to the filtrate, dropwise. The reaction mixture is stirred for one and half hours at room temperature, evaporated <u>in vacuo</u>, and worked up as described in Example 6. The residue is rubbed with ether, filtered and dried. A 1:1 mixture of the $\Delta^2$- and $\Delta^3$-cephem compounds is obtained. The mixture is separated by column chromatography (eluant : acetonitrile ,

carrier : silica gel, Merck Type 7734), to obtain the benzhydryl ester of the heading compound, m.p. 142-146°

1.35 g of this benzhydryl ester are dissolved in 9 ml of anisole and added, dropwise, under argon, and with ice-cooling, to 8 ml of trifluoroacetic acid. After addition, the mixture is stirred at 0° for 1 hour. The reaction mixture is worked up by repeated evaporation with benzene and chloroform, and the residue is rubbed with ether, filtered and dried _in vacuo_. M.p. 132-144°.

EXAMPLE 9: Potassium 6-{α-[1-benzyl-6-methyl-(4)-pyridone-3-carbonylamino]phenylacetamido penicillanate

The heading compound m.p. 255-265° (decomp.) may be obtained in manner analogous to that of Example 5 employing appropriate starting materials in approximately equivalent amounts.

EXAMPLE 10: 6-{α-[1-(2,2-diethoxyethyl)-6-methyl-(4)-pyridone-3-carbonylamino]phenylacetamido - penicillanic acid

The heading compound, m.p. 188°, may be obtained in manner analogous Example 5, employing appropriate starting materials in approximately equivalent amounts.

EXAMPLE 11: Potassium 6-{α-[1-ethyl-6-hydroxy-(4)-pyridone-3-carbonylamino]p-hydroxyphenylacetamido}-penicillanate

7 ml of triethylamine are added to a suspension of 9.3 g of 1-ethyl-6-hydroxy-(4)-pyridone-3-carboxylic acid in dry dichloromethane, and the mixture is stirred for 15 minutes at room temperature to obtain a clear solution. 3.7 ml of thionyl chloride in 20 ml of dry dichloromethane are added at 0°, dropwise, and the mixture is stirred for 3 hours at room temperature. To a suspension of 20.8 g of amoxycillin trihydrate in 500 ml of dry dichloromethane are added, at 0°, successively, 100 ml of dry hexamethylphosphoric acid triamide, 14 ml of dry triethylamine, and 50 g of molecular sieve 3A. After stirring for 3 hours at room temperature, the practically clear solution is filtered and washed with 50 ml of dry dichloromethane. To this solution, are added, dropwise, at -30° and with stirring, simultaneously, the above prepared solution of 1-ethyl-6-hydroxy-(4)-pyridone-3-carboxylic acid chloride and a solution of 10.5 ml of dry triethylamine in 100 ml of dry dichloromethane. After the addition, the cooling bath is removed and the mixture is stirred at room temperature for 2 hours. The mixture is then concentrated on a rotary evaporator and the residue is taken up in 100 ml of ice-water. After addition of 5 g of sodium bicarbonate, the mixture is extracted once with 60 ml of chloroform, the aqueous phase is freed of solvent on a rotary evaporator and acidified with 1N hydrochloric acid, with ice cooling and thorough stirring, to pH 2.5. The precipitate is filtered off, washed with water and dried over phosphorous pentoxide <u>in vacuo</u>, for 30 hours at 30°. For further purification, the residue is dissolved in 100 ml of water and 3.6 g of potassium bicarbonate, extracted 5 times with chloroform and the aqueous phase, after removal of residual solvent, is acidified with 1N hydrochloric acid to pH 2.5. 14 g of the resulting free acid form of the title compound is dissolved in a solution of 2.38 g of potassium bicarbonate in 100 ml of water, the solution is filtered and the filtrate lyophilised. For purification, the potassium salt is washed 5 times with dichloromethane to obtain the heading compound, m.p. > 250° (decomp.).

**EXAMPLE 12:** 7-{[D(-)-α-(1-ethyl-6-methyl-(4)-pyridone-3-carbonylamino)phenylacetamido]}cephalosporanic acid potassium salt

4.23 g of cephaloglycin monohydrate are suspended in 100 ml of dichloromethane in the presence of 5Å molecular sieve. 2.8 ml of triethylamine are added and the mixture

is stirred for 60 minutes at room temperature. The molecular sieve is separated off and the filtrate is employed as solution A.

1.81 g of 1-ethyl-6-methyl-(4)-pyridone-3-carboxylic acid is suspended in 35 ml of dichloromethane, mixed with 1.4 ml of triethylamine, cooled to 0° and mixed, dropwise, with a solution of 0.96 ml of ethyl chloroformate in 4 ml of dichloromethane. After 60 minutes at 0°, this reaction mixture is employed as solution B. Solution A is cooled to -20° and solution B is added, dropwise, thereto. After 12 hours at -20° and 2 hours at 0°, the solvent is evaporated off *in vacuo* and the residue is taken up in 100 ml of water. The aqueous solution is shaken with the same volume of diethylether, separated from somewhat insoluble material and the pH thereof is adjusted to pH 1.5 with 6N hydrochloric acid, whereby the title compound precipitates out in free acid form. The precipitate is filtered off, washed well with water and dried *in vacuo* at room temperature.
M.p. 162°.

For preparation of the potassium salt, the free acid is mixed with an equivalent quantity of potassium bicarbonate in water and the mixture lyophilised.

In manner analogous to the appropriate preceding Examples, employing appropriate starting materials in approximately equivalent amounts, the compounds elucidated in the following Tables I to V may be obtained.

Table I

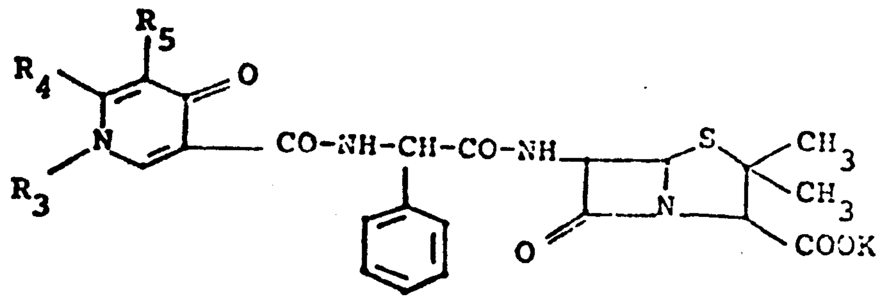

| Ex. | $R_3$ | $R_4$ | $R_5$ | M.P. |
|---|---|---|---|---|
| 13 | $-C_2H_5$ | OH | Br | 245° |
| 14 | n-$C_4H_9$ | $CH_3$ | H | 215-230° |
| 15 | $-C_6H_5$ | OH | H | 270° (decomp.) |
| 16 | $-C_6H_5$ | $CH_3$ | H | 212-215° |
| 17 | 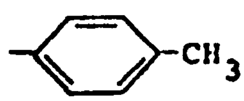 | $CH_3$ | H | 222-240° |
| 18 | 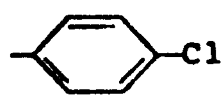 | $CH_3$ | H | >193° (decomp.) |
| 19 | $-C_2H_5$ | $CH_3$ | Br | 229-232° |
| 20 | $-CH_2-C_6H_5$ | $CH_3$ | Br | 228-233° |

| Ex. | $R_3$ | $R_4$ | $R_5$ | M.P. |
|---|---|---|---|---|
| 21 | 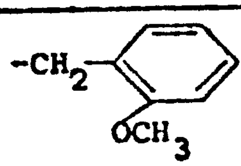 | $CH_3$ | H | 205-208° |
| 22 | 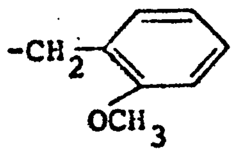 | $CH_3$ | Br | 220-225° |
| 23 | $-CH_2.C_6H_5$ | CN | H | 218-222° |
| 24 | $-CH_2.CH=CH_2$ | $CH_3$ | Br | 221-224° |

Table II

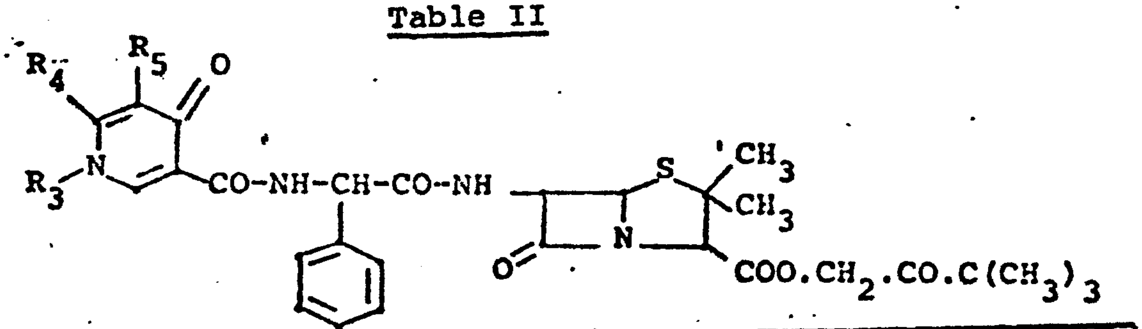

| Ex. | $R_3$ | $R_4$ | $R_5$ | M.P. |
|---|---|---|---|---|
| | 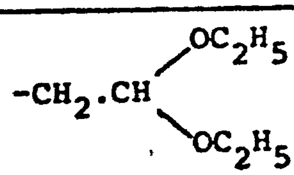 | $CH_3$ | H | from 110° (decomp.) |
| 26 | $-CH_2.C_6H_5$ | $CH_3$ | H | 120° (decomp.) |
| 27 | $-C_6H_5$ | $CH_3$ | H | 121-123° (decomp.) |
| 28 | 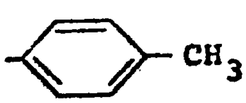 | $CH_3$ | H | 117-119° |

Table III

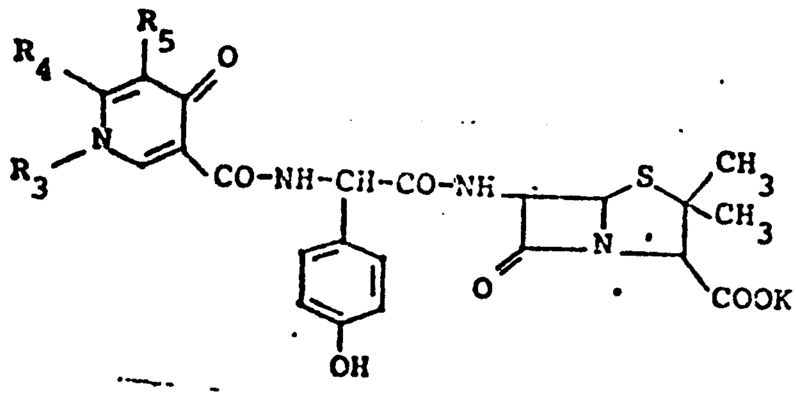

| Ex. | $R_3$ | $R_4$ | $R_5$ | M.P. |
|---|---|---|---|---|
| 29 | $-C_2H_5$ | $CH_3$ | H | 225° (decomp.) |
| 30 | $-CH_2.CH=CH_2$ | $CH_3$ | H | 220° (decomp.) |
| 31 | $-CH_2.CH(OC_2H_5)_2$ | $CH_3$ | H | 210° (decomp.) |
| 32 | $-CH_2.C_6H_5$ | $CH_3$ | H | 210° (decomp.) |
| 33 | $-C_6H_5$ | $CH_3$ | H | 232-235° (decomp.) |
| 34 | 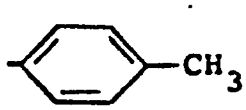 | $CH_3$ | H | 228-230° |
| 35 | 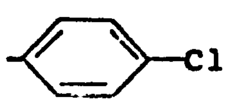 | $CH_3$ | H | 225° (decomp.) |

| Ex. | $R_3$ | $R_4$ | $R_5$ | M.P. |
|---|---|---|---|---|
| 36 | $-CH_2.C_6H_5$ | $CH_3$ | Br | 230-234° |
| 37 | 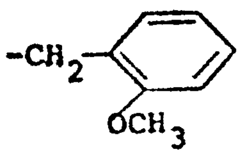 | $CH_3$ | H | 228-234° |
| 38 | 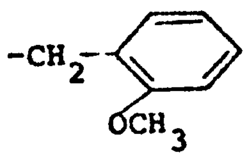 | $CH_3$ | Br | 225-228° |
| 39 | $-CH_2.CH=CH_2$ | $CH_3$ | Br | 238-242° |
| 40 | $-CH_2.C_6H_5$ | H | H | 206-209° |

Table IV

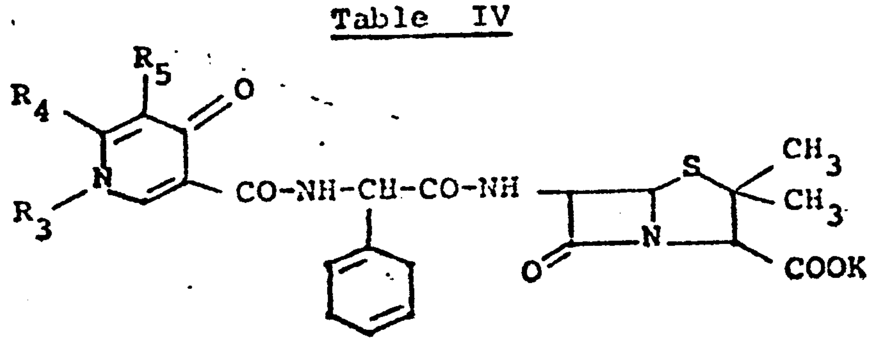

| Ex. | $R_3$ | $R_4$ | $R_5$ | M.P. |
|---|---|---|---|---|
| 41 | $-C_2H_5$ | $CH_3$ | Br | 227-230° |
| 42 | $-CH_2.C_6H_5$ | $CH_3$ | Br | 226-228° |
| 43 | 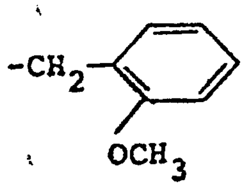 | $CH_3$ | H | 214-217° |

| Ex. | $R_3$ | $R_4$ | $R_5$ | M.P. |
|---|---|---|---|---|
| 44 | 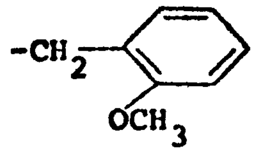 | $CH_3$ | Br | 213-217° |
| 45 | $-CH_2.C_6H_5$ | CN | H | 223-226° |
| 46 | $-CH_2.CH=CH_2$ | $CH_3$ | Br | 225-232° |
| 47 | $-CH_2.C_6H_5$ | H | H | 220-224° |
| 48 | $n-C_4H_9$ | $CH_3$ | H | 212-217° |
| 49 | $-C_2H_5$ | $CH_3$ | H | 226° |
| 50 | $-CH_2.CH=CH_2$ | $CH_3$ | H | 206-208° |
| 51 | $-CH_2.C_6H_5$ | $CH_3$ | H | 209-211° |
| 52 | $-C_6H_5$ | $CH_3$ | H | 219-222° |
| 53 | 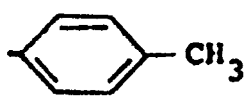 | $CH_3$ | H | 219-221° |
| 54 | $-CH_2-C_6H_5$ | OH | H | 260° (decomp.) |
| 55 | 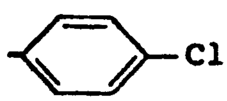 | $CH_3$ | H | 162-175° (decomp.) |
| 56 | $-C_2H_5$ | OH | H | 240° (decomp.) |
| 57 | $-C_2H_5$ | OH | Br | 300° |
| 58 | $-CH_2-C_6H_5$ | OH | H | 262° (decomp.) |

Table V

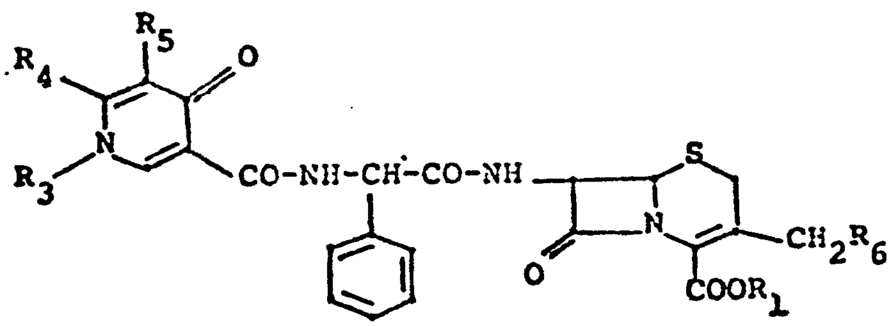

| Ex. | $R_1$ | $R_3$ | $R_4$ | $R_5$ | $R_6$ | M.P. |
|---|---|---|---|---|---|---|
| 59 | K | $-C_2H_5$ | OH | H | H | 246° (decomp.) |
| 60 | K | $-CH_2.CH=CH_2$ | $CH_3$ | H | $-O.CO.CH_3$ | 170° |
| 61 | K | $-C_2H_5$ | OH | H | -"- | 250° (decomp.) |

EXAMPLE 61a: Pivaloyloxymethyl 6-{α-[1-allyl-6-methyl-(4)-pyridon-3-carbonylamino]-p-hydroxyphenyl-acetamido}penicillanate

The above product, m.p. 129 to 131°, may be obtained in manner analogous to the preceding Examples using appropriate starting materials in approximately equivalent amounts.

**EXAMPLE 62:** **Potassium 6-{α-[1-ethyl-6-hydroxy-(4)-pyridone-3-carbonylamino]p-hydroxyphenylacetamido}-penicillanate**

3.3 g of α- 1-ethyl-6-hydroxy-(4)-pyridone-3-carbonylamino -4-hydroxyphenylacetic acid are dissolved in 200 ml of dichloromethane and 3 ml of triethylamine and the mixture is cooled to -30°. After addition of 1.34 ml of isobutyl chloroformate the mixture is stirred for 1 hour at -30°. Finally a solution of N-trimethylsilyl-6-aminopenicillanic acid trimethylsilyl ester (prepared from 2.1 g of 6-APA and 6 g of N,O-bis-trimethylsilyl-acetamide in 100 ml of dichloromethane) is added, dropwise. The reaction mixture is stirred for 3 hours at -15° and for 1 hour at room temperature. The solvent is removed, the residue is taken up in ethyl acetate and mixed with 80 ml of semi-concentrated sodium bicarbonate solution. The organic phase is separated off and the aqueous phase is acidified to pH 2 with dilute hydrochloric acid. The precipitate is filtered off, washed with water and dried to obtain the heading compound, m.p. 225° (decomp.).

The following Examples illustrate the production of starting materials of formula IV:-

a) 1-Ethyl-6-methyl-(4)-pyridone-3-carboxylic acid

1.81 g of 3-dimethylaminomethylene-4-oxo-6-methyl-2-pyrone are dissolved in 2 ml of water and mixed with 4.5 ml of 70% aqueous ethylamine solution. The mixture is held at room temperature for 15 minutes and refluxed for 15 minutes. After cooling, the mixture is evaporated to dryness, the residue is taken up in a little water, and brought to pH 5 with glacial acetic acid, whereby the heading compound crystallises out. The crystals are filtered off and recrystallised from water. M.p. 183-185°.

b) 1-Allyl-6-methyl-(4)-pyridone-3-carboxylic acid

In manner analogous to Example a) above, and employing appropriate starting materials in approximately equivalent amounts, the heading compound, m.p. 115-117° (from water) may be obtained.

c) 1-Benzyl-6-methyl-(4)-pyridone-3-carboxylic acid

1.81 g of 3-dimethylaminomethylene-4-oxo-6-methyl-2-pyron are dissolved in 2ml of water. 2.78 g of benzylamine are added and the mixture is stirred at room temperature for 15 minutes. The mixture is then refluxed for 1 1/2 hours. After evaporation to dryness, the residue

is taken up in a little water, acidified with glacial acetic acid and extracted with ethyl acetate. The ethyl acetate phase is shaken with sodium bicarbonate solution and the aqueous phase acidified to pH 3.5. The resulting precipitate is taken up in ethyl acetate, the organic phase dried and evaporated to dryness. The residue is rubbed with diisopropylether to obtain the heading compound, m.p. 148-150°.

d) 1-(2-Diethoxy)ethyl-6-methyl-(4)-pyridone-3-carboxylic acid

In manner analogous to Example c) and employing appropriate starting materials in approximately equivalent amounts, the heading compound, m.p. 90°, may be obtained.

e) 1-Ethyl-6-hydroxy-(4)-pyridone-3-carboxylic acid

To a suspension of 114.5 g of sodium hydride (80% in oil) in 3 litres of ether are added, dropwise, with stirring, 687 g of acetone dicarboxylic acid diethyl ester. After standing overnight, 200 ml of ethyl isothyocyanate are added over a period of 1 hour and the mixture is then boiled for 2 hours. The mixture is cooled, extracted with 600 ml of water and the aqueous phase is acidified to pH 1 with concentrated hydrochloric acid. Extraction with ethyl

acetate yields, after evaporation of the solvent, 1-ethyl-6-hydroxy-4(1H)-oxo-2-mercaptopyridine-3-carboxylic acid m.p. 100-105°. 126 g of this product are dissolved in 1 litre of 1N caustic soda and the mixture is heated to boiling. To the boiling solution are added, over a period of 2 hours, six 25 g portions of Raney-Nickel. The mixture is filtered, concentrated to a volume of approximately 500 ml and acidified, under ice-cooling, to pH 1 with concentrated hydrochloric acid. The resulting precipitate of the heading compound is filtered off, washed and dried, m.p. 250-255°.

f) 6-Hydroxy-1-phenyl-(4)-pyridone-3-carboxylic acid.

In manner analogous to Example e) and employing appropriate starting materials in approximately equivalent amounts, the heading compound, m.p. 250°, may be obtained.

g) 1-Ethyl-5-bromo-6-methyl-(4)-pyridone-3-carboxylic acid

1 mM of 1-ethyl-6-methyl-(4)-pyridone-3-carboxylic acid ethyl ester is dissolved in 30 ml of dry dichloromethane, mixed with 1.1 mM of N-bromosuccinimide and stirred at room temperature for 12 hours. The solution is shaken with water, sodium bicarbonate and water, dried with magnesium sulphate at room temperature and rotary evaporated.

The residue is mixed with 50 ml of 1N sodium hydroxide and stirred at room temperature for 12 hours. The mixture is acidified, filtered and dried <u>in vacuo</u> at 50°, to obtain the heading compound, m.p. 181-184°.

In manner analogous to Example g) and employing appropriate starting materials in approximately equivalent amounts, the following compounds h) to j) may be obtained.

h) <u>1-Benzyl-5-bromo-6-methyl-(4)-pyridone-3-carboxylic acid</u>
M.p. 228-232°.

i) <u>5-Bromo-1-(2-methoxybenzyl)-6-methyl-(4)-pyridone-3-carboxylic acid</u>
M.p. 257- 260°.

j) <u>1-Allyl-5-bromo-6-methyl-(4)-pyridone-3-carboxylic acid</u>
M.p. 147-150°.

k) <u>1-Benzyl-6-cyano-(4)-pyridone-3-carboxylic acid</u>

1 mM of 1-benzyl-6-methyl-(4)-pyridone-3-carboxylic acid is stirred with 1.1 mM of Meerwein salt(triethyloxonium-tetrafluoroborate) in dichloromethane for 12 hours at room temperature. The solution is carefully shaken with sodium bicarbonate, washed with water and dried over magnesium sulphate. Rotary evaporation of the solvent yields

1-benzyl-6-methyl-(4)-pyridone-3-carboxylic acid ethyl ester. 1 mM of this ester in 20 ml of dry dioxane is mixed with 1.3 mM of selenium dioxide and held at 80° for 12 hours with stirring. After cooling, the solvent is removed by rotary evaporation, the residue is taken up in 50 ml of methanol, 2 spatula tips of active charcoal are added, and the mixture is boiled for 5 minutes. The solution is filtered and evaporated. The residue is taken up in 10 ml of ethanol and slowly added, dropwise, to a solution of 5 mM of hydroxylamine and 30 ml of water. The solution is stirred overnight, filtered, taken up in 50 ml of 1N sodium hydroxide and stirred at room temperature for 12 hours. The solution is carefully filtered, acidified to pH 3 with 2N hydrochloric acid, filtered, dried and crystallised from ethyl acetate/methanol. 1 mM of the resulting oxime (m.p. 238-240°) is mixed with 10 ml of acetic anhydride and warmed for 1 hour at 140°. All volatile components are removed by rotary evaporation, and the residue is recrystallised from toluene, to obtain 1-benzyl-6-cyano-(4)-pyridone-3-carboxylic acid, m.p. 198-200°.

1) <u>1-Benzyl-(4)-pyridone-3-carboxylic acid</u>

1 mM of product k) above is stirred for 12 hours in 30 ml of 1N NaOH at 40°. The solution is carefully filtered, acidified with 2N hydrochloric acid and filtered. 1 mM of the residue (m.p. 202-206°; 3,6-dicarboxylic acid) is warmed

to 190° for 10 minutes whereupon gas bubbles appear. Cooling and crystallisation from toluene yields the heading compound, m.p. 150-152°.

m) 1-Benzyl-6-hydroxy-(4)-pyridone-3-carboxylic acid

1.05 g of 4,6-dihydroxypyridine-3-carboxylic acid ethyl ester are heated, under argon, with 13.1 g of dibenzylphosphite for 6 hours at 150°. The reaction mixture is then divided between water and ethyl acetate, the organic phase is washed with sodium bicarbonate and dried with magnesium sulphate. After evaporation of the solvent, 1-benzyl-4-hydroxy-(6)-pyridone-3-carboxylic acid ethyl ester (m.p. 118°) remains. 2 g of this product are dissolved in 20 ml of methanol and the resulting mixture is mixed with a solution of 1.6 g of sodium hydroxide in 20 ml of water. The mixture is allowed to stand for 22 hours at room temperature, and evaporated *in vacuo*. The residue is taken up in water, extracted with ether, and the aqueous phase is acidified to pH 1 with 6N hydrochloric acid. The precipitate is collected on a suction filter, washed free of hydrochloric acid and dried, to obtain the heading compound, m.p. 235°.

n) <u>1-Ethyl-5-bromo-6-hydroxy-(4)-pyridone-3-carboxylic acid</u>

350 mg of 1-ethyl-6-hydroxy-(4)-pyridone-3-carboxylic acid ethyl ester in 20 ml of chloroform are mixed at room temperature with 265 mg of N-bromosuccinimide. The mixture is evaporated <u>in vacuo</u> and the residue divided between aqueous sodium bicarbonate and dichloromethyl. The organic phase is evaporated, and the residue dissolved in methanol. A 2 molar excess of sodium hydroxide is added and the mixture is evaporated <u>in vacuo</u> for 24 hours. The residue is taken up in water and the pH of the mixture is adjusted to pH 1 with 6N HCl to obtain the heading compound as a crystallised precipitate, m.p. 260°.

o) <u>6-Methyl-1-(4-methylphenyl)-(4)-pyridone-3-carboxylic acid</u>

5.4 g of Dimethylaminomethylene-4-oxo-6-methyl-2-pyrone and 3.2 g of <u>p</u>-toluidine are stirred in 80 ml of dry ethanol for 2 hours at room temperature. 30 ml of 2N sodium ethoxide in ethanol are added and the mixture is heated to boiling for 6 hours. After cooling to room temperature, the solvent is removed, the residue is dissolved in 50 ml of ice-water and the pH of the mixture is adjusted to 1 with concentrated sulphuric acid. The resulting precipitate is filtered, washed with water and

dried to obtain the heading compound, m.p. 209-211°.

In manner analogous to Example n) and employing appropriate starting materials in approximately equivalent amounts, the following compounds may be obtained [p) to s)]:

p) 1-(2-Methoxybenzyl)-6-methyl-(4)-pyridone-3-carboxylic acid

M.p. 168-172°.

q) 1-(n-Butyl)-6-methyl-(4)-pyridone-carboxylic acid

M.p. 130-136°.

r) 6-Methyl-1-phenyl-(4)-pyridone-3-carboxylic acid

M.p. 209-211°.

s) 1-(4-Chlorophenyl)-6-methyl-(4)-pyridone-3-carboxylic acid

M.p. 262-265°.

The following Example illustrates the production of a starting material of formula VI:-

s) D-N-(1-ethyl-6-hydroxy-(4)-pyridone-3-carbonyl)-4-hydroxyphenylglycine

0.37 g of 1-ethyl-6-hydroxy-(4)-pyridone-3-carboxylic acid are dissolved in 15 ml of dichloromethane and 0.28 g of trimethylamine, and the resulting solution mixed at 0° with 0.15 ml of thionyl chloride in 3 ml of dichloromethane.

The resulting mixture is stirred at room temperature for 3 hours and then added, dropwise, at -10°, to a solution of 0.33 g of 4-hydroxyphenylglycine in 15 ml of chloroform and 2.4 ml of N,O-bis-trimethylsilylacetamide. After 3 hours at 0°, 0.84 ml of triethylamine and, 15 minutes after, a solution of 0.16 g of sodium bicarbonate in 15 ml of water are added. The aqueous phase is separated and acidified to pH 1 with 1N hydrochloric acid. The supernatant liquor is decanted off and the residual oil is crystallised from ether to obtain the heading compound as light yellow crystals, m.p. 166-168°.

CLAIMS:

1. Compounds of formula I

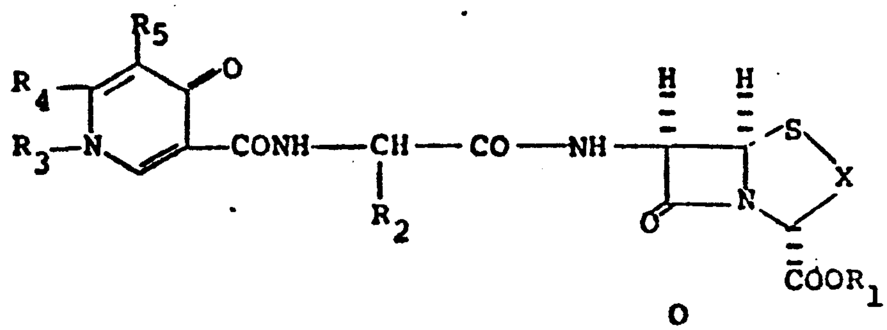

in which $R_1$ is hydrogen or $-CH_2O\overset{O}{\overset{\|}{C}}C(CH_3)_3$,

$R_2$ is phenyl, 4-hydroxyphenyl or 1,4-cyclohexadien-1-yl,

$R_3$ is lower alkyl, unsubstituted or mono- or poly-substituted by alkoxy; allyl; cyclohexyl; cyclopropylmethyl; or benzyl or phenyl, such benzyl or phenyl being unsubstituted or mono- or poly-substituted in the phenyl ring by lower alkyl, lower alkoxy, lower alkoxycarbonyl, cyano, trifluoromethyl, chlorine or bromine,

$R_4$ is hydrogen, cyano, lower alkyl or hydroxy,

$R_5$ is hydrogen, chlorine or bromine,

and X is a group of formula IIa or IIb,

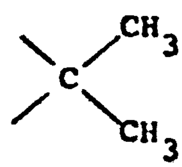

IIa

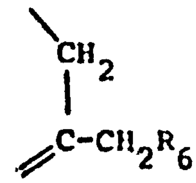

IIb

in which $R_6$ is hydrogen or acetoxy,

and pharmaceutically acceptable salts of compounds of formula I, in which $R_1$ is hydrogen.

2. The compound of Claim 1, which is 6-{α-[1-ethyl-6-hydroxy-(4)-pyridone-3-carbonylamino]} p-hydroxyphenyl-acetamido penicillanic acid, or a pharmaceutically acceptable salt thereof.

3. The compound of Claim 1, which is potassium 6-{α-[1-ethyl-6-hydroxy-(4)-pyridone-3-carbonylamino]p-hydroxyphenylacetamido} penicillanate.

4. A process for the production of the compound of formula I, stated in Claim 1, characterised by

a) reacting a compound of formula III,

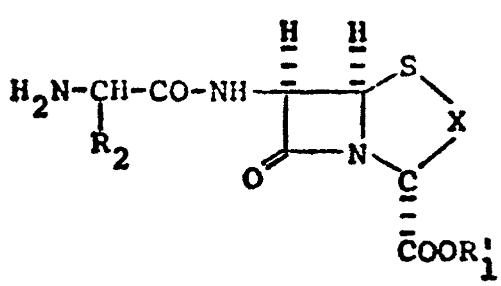

III

in which $R_2$ and X are as defined in Claim 1, and

$R_1'$ is hydrogen, $-CH_2O\overset{O}{\overset{\|}{C}}C(CH_3)_3$, or a carboxylic acid protecting group removable under mild conditions,

with a carboxylic acid of formula IV,

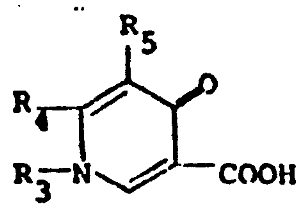

IV

in which $R_3$, $R_4$ and $R_5$ are as defined in Claim 1, or a reactive derivative thereof, and, where $R_1'$ signifies a carboxylic acid protecting group, removing this under mild conditions,

or b) reacting a compound of formula V,

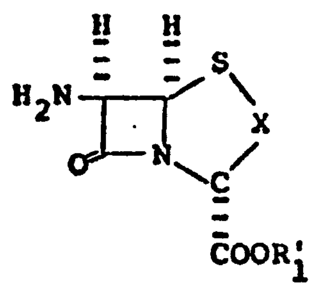

V

in which $R_1'$ and X are as defined above, with a carboxylic acid of formula VI,

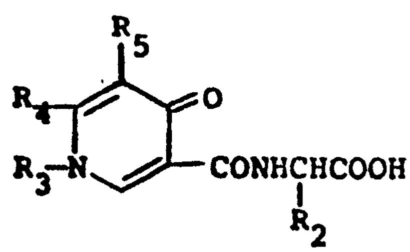

VI

in which $R_2$, $R_3$, $R_4$ and $R_5$ are as defined in Claim 1,

or a reactive derivative thereof,

and, where $R_1'$ signifies a carboxylic acid protecting group, removing this under mild conditions.

5. A pharmaceutical composition comprising an effective amount of the compound of Claim 1, in association with a pharmaceutically acceptable diluent or carrier.

6. Compounds of formula IV, stated in Claim 4.

7. Compounds of formula VI, stated in Claim 4.

3700/MO/HD